Europäisches Patentamt

European Patent Office

Office européen des brevets

(11) Publication number: **0 159 866 B1**

# EUROPEAN PATENT SPECIFICATION

(45) Date of publication of patent specification: **03.04.91**

(21) Application number: **85302548.4**

(22) Date of filing: **11.04.85**

(51) Int. Cl.5: **C12P 13/22**, C12P 13/04, C12N 1/20, //(C12P13/22, C12R1:00),(C12P13/04, C12R1:06),(C12N1/20, C12R1:06)

(54) Process for production of L-amino acids.

(30) Priority: **11.04.84 JP 70906/84**
**25.06.84 JP 129261/84**

(43) Date of publication of application:
**30.10.85 Bulletin 85/44**

(45) Publication of the grant of the patent:
**03.04.91 Bulletin 91/14**

(84) Designated Contracting States:
**DE FR GB IT NL SE**

(56) References cited:
**FR-A- 2 393 848**
**FR-A- 2 431 536**
**GB-A- 1 514 837**
**GB-A- 2 042 531**

**BULLETIN DE LA SOCIETE CHIMIQUE DE FRANCE, vol. II, 1980, pages 91-95; M. GUIVARCH et al.: "Obtention d'amino acides optiquement actifs à l'aide d'hydantoinases"**

(73) Proprietor: **DENKI KAGAKU KOGYO KABUSHIKI KAISHA**
**4-1, Yuraku-cho 1-chome**
**Chiyoda-ku Tokyo(JP)**

(72) Inventor: **Miyoshi, Teruzo**
**4-4-12 Ogawa**
**Machida-shi Tokyo(JP)**
Inventor: **Kitagawa, Hironoshin**
**1-36-203 Fujinodai Danchi 3486 Honmachida**
**Machida-shi Tokyo(JP)**
Inventor: **Kato, Masaaki**
**Ho-25-511 Machida Kiso Jutaku 2577 Honmachida**
**Machida-shi Tokyo(JP)**
Inventor: **Chiba, Susumu DENKI KAGAKU KOGYO K.K.**
**Chuo Kenkyusho Machida-Ryo 5-25-18 Morino**
**Machida-shi Tokyo(JP)**

DERWENT JAPANESE PATENTS REPORT, vol. 6, no. 31, 11th September 1967, page 2, Derwent Publications LTD, London, GB; & JP-A-1 385 067 (AJINOMOTO CO. LTD) 05-08-1967

(74) Representative: **Woods, Geoffrey Corlett et al J.A. KEMP & CO. 14 South Square Gray's Inn London WC1R 5EU(GB)**

**Description**

This invention relates to a microbial process for the production of L-amino acids, especially from 5-substituted hydantoins or N-carbamyl derivatives of amino acids using microbial enzymes.

There have been proposed microbial processes for producing L-amino acids from 5-substituted hydantoins using various microbial enzyme Sources (see JP-B-l3850/67, JP-B-2274/79 and JP-B-8749/79). However, these processes are unsatisfactory from the efficiency viewpoint.

There has also been known a process for producing L-methionine from DL-N-carbamylmethionine using a microbial enzyme (see JP-B-29678/80). This process requires separation of L-methionine from D-N-carbamylmethionine which remains in the reaction mixture, and accordingly is not sufficiently effective.

The present inventors have now found that certain microbial strains belonging to the genus Arthrobacter are capable of effectively converting 5-substituted hydantoins into L-amino acids.

The present invention provides a process for producing an L-amino acid in which a 5-substituted hydantoin or an N-carbamyl derivative of an amino acid is treated with cells or the contents of cells of a strain which belongs to the genus Arthrobacter and which is capable of converting the 5-substituted hydantoin or N-carbamyl derivative into a corresponding L-amino acid, characterised in that the strain is Arthrobacter sp. DK-200 [FERM P-7472 (FERM BP-730)] or a functionally equivalent mutant or a functionally equivalent genetically engineered variant thereof.

The present invention also provides a culture in a culture medium, comprising a source of assimilable carbon, a source of assimilable nitrogen and inorganic salts of a strain belonging to the genus Arthrobacter and being capable of converting a 5-substituted hydantoin or N-carbamyl derivative of an amino acid into a corresponding L-amino acid, the culture being substantially free of other microorganisms, characterized in that the strain is Arthrobacter sp. DK-200 [FERM P-7472 (FERM BP-730)] or a functionally equivalent mutant or functionally equivalent genetically engineered variant thereof.

The present invention further provides a strain belonging to the genus Arthrobacter and being capable of converting a 5-substituted hydantoin or N-carbamyl derivative of an amino acid into a corresponding L-amino acid, characterized in that the strain is Arthrobacter sp. DK-200 [FERM P-7472 (FERM BP-730)] or a functionally equivalent mutant or functionally equivalent genetically engineered variant thereof.

The Arthrobacter strain is capable of converting 5-substituted hydantoins and/or N-carbamyl derivatives of L-, D- or DL-amino acids into the corresponding L-amino acids.

Arthrobacter sp. DK-200 has been isolated by the present inventors.

The strain Arthrobacter sp. DK-200 was deposited on February 27, l984 at the Fermentation Research Institute, Agency of Industrial Science and Technology, Ministry of International Trade and Industry, l-3, Higashi l-chome Yatabe-machi Tsukuba-gun Ibaraki-ken 305, JAPAN, and was assigned the FERM P accession number 7472, which corresponds to FERM BP-730.

The taxonomical characteristics of the strain Arthrobacter sp. DK-200, FERM P-7472 (FERM BP-730), are as follows:

l. Morphological properties:

0.3-0.5 x 0.8-5.0 micrometers cell size, polymorphic, not motile, no flagellum, no spore, negative or slightly positive in Gram staining but having gram-positive granules, and not acid-fast;

2. Growth properties in culture media:

Nutrient agar plate culture; pale white, circular, convex and entire colonies; Nutrient agar slant culture; moderate growth, glossy, and filamentous; Nutrient liquid culture; uniformly turbid, precipitating, and no particular growth on the liquid surface; Nutrient gelatin stab culture; liquefying;

3. Physiological properties: nitrates not reduced, positive in denitrification, negative in MR test, negative in VP test, indole not produced, hydrogen sulfide not produced, starch not hydrolyzed, citric acid not utilized in either Koser medium nor Christensen medium, both nitrates and ammonium salts utilized, pigment not produced, negative in urease activity, negative in oxidase activity, positive in catalase activity, aerobic, growth at a temperature in the range of l7.8-33.2 $^\circ$ C and a pH in the range of 5-l0, oxidative in OF test, lysine as dibasic amino acid in cell wall and not containing meso-diaminopimelic acid and arabinose, positive in DNase activity, positive in casein-hydrolysis, and growth on 2% NaCl and weak growth on 5% NaCl;

Generation of acid and gas from sugars:

| Sugar | Acid | Gas |
|---|---|---|
| L-arabinose | − | − |
| D-xylose | + | − |
| D-glucose | ± | − |
| D-mannose | + | − |
| D-fructose | ± | − |
| D-galactose | ± | − |
| maltose | ± | − |
| sucrose | ± | − |
| lactose | ± | − |
| trehalose | ± | − |
| D-sorbitol | ± | − |
| D-mannitol | ± | − |
| inositol | ± | − |
| glycerin | + | − |

Assimilation of hydrocarbons;

| | |
|---|---|
| p-hydroxybenzoic acid | − |
| gluconic acid | − |
| lactic acid | − |
| acetic acid | − |
| glucose | + |
| sucrose | + |
| xylose | + |
| lactose | − |
| mannitol | + |

According to Bergey's Manual of Determinative Bacteriology, 8th Ed. (l974), strain DK-200 belongs to the genus Arthrobacter on the basis of its taxonomical characteristics: that is, (l) polymorphic, (2) gram-positive granules contained in the cell, and (3) lysine contained in the cell wall and no meso-diaminopimelic

4

EP 0 159 866 B1

acid nor arabinose contained in the cell wall.

The strain DK-200 of the invention belongs to new species of the genus Arthrobacter since its taxonomical properties are distinct from those of any known species.

The strain DK-200 may be prepared in large quantities by any conventional aerobic liquid culture in a medium containing a carbon source, a nitrogen source, inorganic salts and a small amount of organic nutrients which can be assimilated by the strain. The enzyme activity can be enhanced by an addition of a small amount of for example L-carbamyltryptophan to the culture medium. The culture may be carried out at a pH of from 4-II and a temperature of from I5-40°C for a time of from 5-I20 hours.

The thus obtained culture containing the cells is preferably used in the invention. In addition there can also be utilized treated materials of the cells, such as frozen cells, lyophilized cells, sonicated cells, and cell debris, e.g. homogenates and extracts of the cells, as well as intact cells. Furthermore, any variant having improved activity by some variation treatment can of course be used effectively in the present invention.

Examples of 5-substituted hydantoins which can be used as starting materials in the invention include 5-benzylhydantoin, 5-indolylmethylhydantoin and 5-(4-hydroxybenzyl)-hydantoin. Examples of N-carbamyl derivatives of amino acids include N-carbamylphenylalanine, N-carbamyltryptophan and N-carbamyltyrosine. These starting materials may be in D, L or DL from. The starting materials are known or may be prepared in any conventional manner. For example, DL-N-carbamyl derivatives may be prepared by the reaction of DL-amino acids with potassium cyanide, or by the hydrolysis of DL-hydantoin derivatives of amino acids under appropriate conditions.

In the process of the invention, there is preferably used 0.I-40%, more preferably I-I0% by weight of the 5-substituted hydantoin or preferably 0.I-50% by weight of the N-carbamyl derivative of an amino acid in the reaction mixture. The reaction of the invention may be conveniently carried out at a pH of from 4 to II, preferably from 6 to 9. Reaction conditions outside these ranges are inefficient in practice in view of the enzyme activity and stability. The pH may vary as the reaction proceeds and, accordingly, the pH should be maintained at a desired value by the addition of an appropriate neutralizing agent. The reaction temperature may be from I5 to 50°C. In addition, a small amount. e.g. 0.I-I0 mM, of a metal ion, provided by compounds such as $MnCl_2$ and $CoCl_2$ will give better results.

The L-amino acid produced can be separated from the reaction mixture, for example by centrifuging the mixture to remove insolubles such as the cells, passing the supernatant through a strong acid-$H^+$ type cation-exchange resin to adsorb the L-amino acids followed by elution with aqueous ammonia, and further concentrating and cooling to deposit crystals.

According to the present invention, L-amino acids can be produced very efficiently from 5-substituted hydantoins or N-carbamyl derivatives of amino acids, irrespective of the D-, L- or DL-form of the starting materials.

The invention is now further described in the following Examples. In these Examples, the percentages are by weight unless otherwise noted.

EXAMPLE I :

A medium containing 0.5% glucose, 0.2% yeast extract, 0.I% disodium hydrogenphosphate, 0.05% potassium dihydrogenphosphate and 0.05% magnesium sulfate, having a pH of 7.5, was distributed into a number of test tubes and sterilized at I20°C for I5 minutes. DL-N-carbamyltryptophan which had also been sterilized was added to each test tube to provide a final concentration of 0.05%. Arthrobacter sp. DK-200 was then inoculated into the medium and cultured while shaking at 30°C for 24 hours.

To the cultured medium there was added each of the 5-substituted hydantoins listed in Table I below. Shaking of the culture was further continued at 30°C for 24 hours. After reaction the cells were removed by centrifugation. Amino acids in the supernatants were quantitatively analyzed by HPLC and a bioassay using Lactobacillus arabinosus (ATCC No. 80I4). Based on the results of both analyses, each product was identified as the L-amino acids shown in Table I.

5

## Table 1

| 5-Substituted Hydantoin (0.1 M) | Product L-Amino acid | HPLC (mM) | Bioassay (mM) |
|---|---|---|---|
| DL-5-benzylhydantoin | L-phenylalanine | 58 | 58 |
| DL-5-indolylmethyl-hydantoin | L-tryptophan | 61 | 61 |
| DL-5-(4-hydroxy-benzyl)hydantoin | L-tyrosine | 55 | 55 |

EXAMPLE 2 :

Into a 500 ml flask there was added 100 ml of a medium containing 0.5% glucose, 0.2% yeast extract, 0.l% disodium hydrogenphosphate, 0.05% potassium dihydrogenphosphate, 0.05% magnesium sulfate and 0.l% DL-5-indolylmethylhydantoin, having a pH of 7.5. The flask was sterilized at l20−C for l5 minutes. To the flask there was inoculated 5 ml of a seed culture of Arthrobacter sp. DK-200 which had been cultured at 30°C for 20 hours in a medium, having a pH of 7.5, containing 0.5% glucose, 0.5% yeast extract, 0.5% polypeptone and 0.5% sodium chloride. After 24 hour culture at 30°C, the cells were separated from the culture medium by centrifugation and washed once with saline.

The cells were suspended in l00 ml of 0.l M Tris HCl buffer, having a pH of 7.0, containing 0.2 M DL-5-benzylhydantoin and 0.5 mM cobalt chloride, and incubated at 35°C for 65 hours. After reaction the cells were removed by centrifugation. The supernatant was concentrated to 20 ml and cooled to deposit crystals. The crystals were analyzed by TLC, HPLC, NMR and polarimetry and data corresponding to a standard specimen of L-phenylalanine were obtained as shown in Table 2.

## Table 2

| L-phenylalanine | $[\alpha]_D^{20}$ (c=2, $H_2O$) |
|---|---|
| Product of Example 2 | −34.6 |
| Standard Specimen | −34.7 |

EXAMPLE 3 :

Cells obtained in the same manner as Example l were suspended in 2 ml of 0.l M Tris HCl buffer, having a pH of 7.5, containing 0.5 mM cobalt chloride and l mM manganese chloride. The suspension was subjected three times to ultrasonication at 20 kHz for 3 minutes while cooling in an ice bath.

D-5-benzylhydantoin was added (0.l M) to the suspension containing the cell debris, which was then incubated at 35°C for 40 hours. After reaction insolubles were centrifuged out, L-phenylalanine was obtained from the supernatant with a yield of 82%. Identities of the product were confirmed by HPLC and bioassay.

EXAMPLE 4 :

A medium, having a pH of 7.0, containing 0.5% glucose, 0.2% yeast extract, 0.1% disodium hydrogen-phosphate, 0.05% potassium dihydrogenphosphate and 0.05% magnesium sulfate, was distributed into a number of test tubes in an amount of 5 ml in each test tube, and sterilized at 120°C for 15 minutes. DL-N-carbamyltryptophan, which had also been sterilized, was added to each of the tubes to provide a final concentration of 0.1%. Arthrobacter sp. DK-200 was then inoculated into the medium and incubated at 30°C for 24 hours with shaking.

Cells collected from the medium by centrifugation were washed with the same amount of saline and suspended in 5 ml of 0.1 M Tris HCl buffer, having a pH of 7.5, containing 0.1 mM manganese chloride and 0.1 M L-N-carbamylamino acid, each of which is listed in Table 3 below. The reaction was carried out at 35°C for 20 hours. Quantitative measurements of the products by bioassay with Lactobacillus arabinosus , ATCC No. 8014, and HPLC were performed and the results are shown in Table 3. As shown in Table 3, the obtained products were identified as the corresponding L-amino acids.

## Table 3

| Substrate (0.1 M) | Product L-amino acid | HPLC (mM) | Bioassay (mM) |
|---|---|---|---|
| L-N-carbamylphenylalanine | L-phenylalanine | 85 | 85 |
| L-N-carbamyltryptophan | L-tryptophan | 79 | 79 |
| L-N-carbamyltyrosine | L-tyrosine | 84 | 84 |

EXAMPLE 5 :

Cells which had been obtained by the same manner as in Example 4 except for using 500 ml Erlenmeyer flasks containing 100 ml of the medium were suspended in 100 ml of 0.1 M Tris HCl buffer, having a pH of 7.0, containing 0.1 M D-N-carbamylamino acid listed in Table 4 below, 0.5 mM cobalt chloride and 1 mM manganese chloride, and incubated at 35°C for 60 hours. HPLC analysis of the reaction mixture showed that the reaction had been almost completed in this procedure.

After centrifugation to remove the cells, the supernatant was concentrated to 25 ml, and about 5 ml ethanol was added and cooled to 5°C to deposit crystals. The crystals were recrystallized and analyzed by HPLC, TLC, bioassay, NMR and polarimetry. Comparisons of the obtained data with those of standard specimens of the corresponding L-amino acids confirmed the identities of the products shown in Table 4.

## Table 4

| Substrate (0.1 M) | Product L-amino acid | Crystal (g) | Specific Rotation $[\alpha]_D^{20}$ ($H_2O$) |
|---|---|---|---|
| D-N-carbamyl-phenylalanine | L-phenylalanine | 1.2 | -34.4 * ( -34.5) |
| D-N-carbamyltryptophan | L-tryptophan | 0.9 | -33.6 * ( -33.7) |
| D-N-carbamyltyrosine | L-tyrosine | 0.7 | -11.8 ** * ( -11.8) |

\*     standard specimen

\*\*     in 5M HCl solution

EXAMPLE 6 :

The procedures of Example 5 were repeated except that D-N-carbamylamino acids were replaced by DL-N-carbamylamino acids. The analysis results of the obtained products corresponded with those of standard specimens, as shown in Table 5.

## Table 5

| Substrate (0.1 M) | Product L-amino acid | Crystal (g) | Specific Rotation $[\alpha]_D^{20}$ ($H_2O$) |
|---|---|---|---|
| DL-N-carbamyl-phenylalanine | L-phenylalanine | 1.7 | -34.4 * (-34.5) |
| DL-N-carbamyl-tryptophan | L-tryptophan | 1.3 | -33.6 * (-33.7) |
| DL-N-carbamyl-tyrosine | L-tyrosine | 1.4 | -11.8 ** * (-11.8) |

\* standard specimen     \*\* in 5M HCl solution

EXAMPLE 7 :

Cells obtained in the same manner as in Example 5 were suspended in 20 ml of 0.l M Tris HCl buffer, having a pH of 7.0, containing 0.5 mM cobalt chloride and l mM manganese chloride. The suspension was subjected three times to ultrasonication at 20 kHz for 3 minutes under cooling in an ice bath.

DL-N-carbamylamino acid was added to the cell debris solution in a concentration of 0.l M and incubated at 30°C for 20 hours. After reaction insolubles were removed by centrifugation, the resulting supernatant was analyzed by HPLC and bioassay. The products were identified as corresponding L-amino acids on the basis of the results of the analyses shown in Table 6.

### Table 6

| Substrate (0.1 M) | Product L-amino acid | HPLC (mM) | Bioassay (mM) |
|---|---|---|---|
| DL-N-carbamylphenylalanine | L-phenylalanine | 78 | 78 |
| DL-N-carbamyltryptophan | L-tryptophan | 75 | 75 |
| DL-N-carbamyltyrosine | L-tyrosine | 69 | 69 |

## Claims

1. A process for producing an L-amino acid in which a 5-substituted hydantoin or an N-carbamyl derivative of an amino acid is treated with cells or the contents of cells of a strain which belongs to the genus Arthrobacter and which is capable of converting the 5-substituted hydantoin or N-carbamyl derivative into a corresponding L-amino acid, characterized in that the strain is Arthrobacter sp. DK-200 [FERM P-7472 (FERM BP-730)] or a functionally equivalent mutant or functionally equivalent genetically engineered variant thereof.

2. A process according to claim I, wherein the 5-substituted hydantoin is L-, D- or DL-5-benzylhydantoin and the L-amino acid is L-phenylalanine.

3. A process according to claim I, wherein the 5-substituted hydantoin is L-, D- or DL-5-indolylmethylhydantoin and the L-amino acid is L-tryptophan.

4. A process according to claim I, wherein the 5-substituted hydantoin is L-, D- or DL-5-(4-hydroxybenzyl)-hydantoin and the L-amino acid is L-tyrosine.

5. A process according to claim I, wherein the N-carbamyl derivative is L-, D- or DL-N-carbamylphenylalanine and the L-amino acid is L-phenylalanine.

6. A process according to claim I, wherein the N-carbamyl derivative is L-, D- or DL-N-carbamyltryptophan and the L-amino acid is L-tryptophan.

7. A process according to claim I, wherein the N-carbamyl derivative is L-, D- or DL-N-carbamyltyrosine and the L-amino acid is L-tyrosine.

8. A process according to any one of the preceding claims, wherein the cells are intact cells or disrupted cells.

9. A process according to claim 8, wherein the intact cells are in culture.

10. A process according to any one of claims I to 7 wherein the cells or the contents of cells are sonicated cells, frozen cells, lyophilized cells or a homogenate or extract derived from the cells.

11. A process according to any one of Claims I to 7, wherein the 5-substituted hydantoin or N-carbamyl derivative is contacted with a culture of the said strain.

12. A culture in a culture medium, comprising a source of assimilable carbon, a source of assimilable nitrogen and inorganic salts of a strain belonging to the genus Arthrobacter and being capable of converting a 5-substituted hydantoin or N-carbamyl derivative of an amino acid into a corresponding L-amino acid, the culture being substantially free of other microorganisms, characterized in that the strain is Arthrobacter sp. DK-200 [FERM P-7472 (FERM BP-730)] or a functionally equivalent mutant or functionally equivalent genetically engineered variant thereof.

13. A strain belonging to the genus Arthrobacter and being capable of converting a 5-substituted hydantoin or N-carbamyl derivative of an amino acid into a corresponding L-amino acid, characterised in that the strain is Arthrobacter sp. DK-200 [FERM P-7472 (FERM BP-730)] or a functionally equivalent mutant or functionally equivalent genetically engineered variant thereof.


## Revendications

1. Un procédé pour la production d'un L-aminoacide, dans lequel une hydantoïne substituée en position 5 ou un dérivé de N-carbamylé d'acide aminé est traité avec des cellules ou le contenu cellulaire d'une souche appartenant au genre Arthrobacter , laquelle est capable de convertir l'hydantoïne substituée en position 5 ou le dérivé N-carbamylé d aminoacide en L-aminoacide, caractérisé en ce que la souche est l'Arthrobacter sp. DK-200 [FERM P-7472 (FERM BP-730)] ou un mutant fonctionnellement équivalent ou un variant obtenu à partir de cette souche par génie génétique et fonctionnellement équivalent.

2. Un procédé selon la revendication I, pour lequel l'hydantoïne substituée en position 5 est la L-D-ou DL-benzylhydantoïne et le L-aminoacide est la L-phénylalanine.

3. Un procédé selon la revendication I, pour lequel l' hydantoïne substituée en position 5 est la L-, D- ou DL-indolyl-méthylhydantoïne et le L-aminoacide est le L-tryptophane.

4. Un procédé selon la revendication I, pour lequel l'hydantoïne substituée en position 5 est la L-, D- ou DL-5-(4-hydroxybenzyl)-hydantoïne et le L-aminoacide est la L-tyrosine.

5. Un procédé selon la revendication I, pour lequel le dérivé N-carbamylé est la L-, D- ou DL-N-carbamylphénylalanine et le L-aminoacide est la L-phénylalanine.

6. Un procédé selon la revendication I, pour lequel le dérivé N-carbamylé est le L-, D- ou DL-N-carbamyltryptophane et le L-aminoacide est le L-tryptophane.

7. Un procédé selon la revendication I, pour lequel le dérivé N-carbamylé est la L-, D- ou DL-N-carbamyltyrosine et le L-aminoacide est la L-tyrosine.

8. Un procédé selon l'une quelconque des revendications précédentes, dans lequel les cellules sont intactes ou disloquées.

9. Un procédé selon la revendication 8 pour lequel les cellules intactes sont en culture.

10. Un procédé selon l'une quelconque des revendications de I à 7, dans lequel les cellules sont des cellules soniquées, des cellules congelées, des cellules lyophilisées, ou un homogénat ou un extrait obtenu à partir de ces cellules.

11. Un procédé selon l'une quelconque des revendications de I à 7, dans lequel l'hydantoïne substituée en position 5 ou le dérivé N-carbamylé est mis en contact avec une culture de ladite souche.

12. Une culture dans un milieu de culture, comprenant une source de carbone assimilable, une source d'azote assimilable et des sels inorganiques, d'une souche appartenant au genre Arthrobacter , capable de convertir une hydantoïne substituée en position 5 ou un dérivé N-carbamylé d'aminoacide en L-aminoacide correspondant, la culture étant pratiquement exempte d'autres microorganismes, caractérisée en ce que cette souche est l'Arthrobacter sp. DK-200 [FERM P-7472 (FERM BP-730)] ou un mutant fonctionnellement équivalent ou un variant de cette souche obtenue par génie génétique et fonctionnellement équivalente.

13. Une souche appartenant au genre Arthrobacter , capable de convertir une hydantoïne substituée en position 5 ou un dérivé N-carbamylé d'aminoacide en L-aminoacide correspondant, caractérisée en ce que cette souche est l'Arthrobacter . sp. DK-200 [FERM P-7472 (FERM BP-730)] ou un mutant fonctionnellement équivalent ou un variant de cette souche obtenu par génie génétique et fonctionnellement. équivalent.

## Ansprüche

1. Verfahren zur Herstellung einer L-Aminosäure, bei dem ein 5-substituiertes Hydantoin oder ein N-Carbamylderivat einer Aminosäure mit Zellen oder den Zellinhalten eines Stammes behandelt wird, der zu der Art Arthrobacter gehört und der in der Lage ist, das 5-substituierte Hydantoin oder N-Carbamylderivat in eine entsprechende L-Aminosäure umzuwandeln, dadurch **gekennzeichnet, dass** der Stamm Arthrobacter sp. DK-200 [FERM P-7472 (FERM BP-730)] oder eine funktionell äquivalente Mutante oder eine funktionell äquivalente, genetisch behandelte Variante davon ist.

2. Verfahren nach Anspruch I, dadurch **gekennzeichnet,** dass das 5-substituierte Hydantoin L-, D- oder DL-5-Benzylhydantoin und die L-Aminosäure L-Phenylalanin ist.

3. Verfahren nach Anspruch I, dadurch **gekennzeichnet,** dass das 5-substituierte Hydantoin L-, D- oder DL-5-Indolylmethylhydantoin und die L-Aminosäure L-Tryptophan ist.

4. Verfahren nach Anspruch I, dadurch **gekennzeichnet,** dass das 5-substituierte Hydantoin L-, D- oder DL-5-(4-Hydroxybenzyl)-hydantoin und die L-Aminosäure L-Tyrosin ist.

5. Verfahren nach Anspruch I. dadurch **gekennzeichnet,** dass das N-Carbamylderivat L-, D- oder DL-N-Carbamylphenylalanin und die L-Aminosäure L-Phenylalanin ist.

6. Verfahren nach Anspruch I, dadurch **gekennzeichnet,** dass das N-Carbamylderivat L-, D- oder DL-N-Carbamyltryptophan und die L-Aminosäure L-Tryptophan ist.

7. Verfahren nach Anspruch I, dadurch **gekennzeichnet,** dass das N-Carbamylderivat L-, D- oder DL-N-Carbamyltyrosin und die L-Aminosäure L-Tyrosin ist.

8. Verfahren nach einem der vorhergehenden Ansprüche. dadurch **gekennzeichnet,** dass die Zellen intakte Zellen oder zertrümmerte Zellen sind.

9. Verfahren nach Anspruch 8, dadurch **gekennzeichnet,** dass die intakten Zellen in Kultur vorliegen.

10. Verfahren nach einem der Ansprüche I bis 7, dadurch **gekennzeichnet,** dass die Zellen oder die Zellinhalte beschallte Zellen, gefrorene Zellen, lyophilisierte Zellen oder ein Homogenat oder Extrakt, das sich von den Zellen ableitet, darstellen.

11. Verfahren nach einem der Ansprüche I bis 7I dadurch **gekennzeichnet,** dass das 5-substituierte Hydantoin oder N-Carbamylderivat mit einer Kultur des Stammes in Kontakt gebracht wird.

12. Kultur in einem Kulturmedium, mit einer Quelle aus assimilierbarem Kohlenstoff, einer Quelle aus assimilierbarem Stickstoff und anorganischen Salzen eines Stammes, der zur Art Arthrobacter gehört und der in der Lage ist, ein 5-substituiertes Hydantoin oder N-Carbamylderivat einer Aminosäure in eine entsprechende L-Aminosäure umzuwandeln, wobei die Kultur im wesentlichen frei von anderen Mikroorganismen ist, dadurch **gekennzeichnet,** dass der Stam Arthrobacter sp. DK-200 [FERM P-7472 (FERM BP-730)] oder eine funktionell äquivalente Mutante oder eine funktionell äquivalente, genetisch behandelte Variante davon ist.

13. Stamm, der zur Art Arthrobacter gehört und in der Lage ist, ein 5-substituiertes Hydantoin oder N-Carbamoylderivat einer Aminosäure in eine entsprechende L-Aminosäure umzuwandeln, dadurch **gekennzeichnet,** dass der Stamm Arthrobacter sp. DK-200 [FERM P-7472 (FERM BP-730)] oder eine funktionell äquivalente Mutante oder eine funktionell äquivalente, genetisch behandelte Variante davon ist.